# EUROPÄISCHE PATENTSCHRIFT

(11) **EP 2 785 824 B1**
(45) Veröffentlichungstag und Bekanntmachung des Hinweises auf die Patenterteilung: **16.12.2020**
(21) Anmeldenummer: 12772236.1
(22) Anmeldetag: 10.10.2012
(51) Int. Cl.: C12M 1/12

(54) **SYSTEM UND VERFAHREN ZUR ZELLABTRENNUNG**
SYSTEM AND METHOD FOR CELL SEPARATION
SYSTÈME ET PROCÉDÉ DE SÉPARATION DE CELLULES

(30) Priorität: 01.12.2011 DE 102011119816
(43) Veröffentlichungstag der Anmeldung: 08.10.2014
(73) Patentinhaber: Sartorius Stedim Biotech GmbH, 37079 Göttingen (DE)
(72) Erfinder: DIEL, Bernhard, 37127 Dransfeld (DE)
(74) Vertreter: Vigand, Philippe
(86) Internationale Anmeldenummer: PCT/EP2012/004236
(87) Internationale Veröffentlichungsnummer: WO 2013/079136

(56) Entgegenhaltungen:
- WO-A1-00/24918
- WO-A1-2010/078404
- DE-A1- 10 017 256
- US-A- 2 455 130
- US-A1- 2004 245 124

## Beschreibung

Die Erfindung betrifft ein Zellabtrennungssystem mit einem Filter und mit einem in einem Rezirkulationskreislauf angeordneten Pulverbeutel, der mit einem pulverförmigen Filterhilfsmittel teilgefüllt ist und dem aus einem Bioreaktor über eine im Rezirkulationskreislauf angeordnete Pumpe eine die abzutrennenden Zellen enthaltende Zelllösung zuführbar ist.
Die Erfindung betrifft weiterhin ein Verfahren zur Zellabtrennung über einen Filter mit einem in einem Rezirkulationskreislauf angeordneten Pulverbeutel, der mit einem pulverförmigen Filterhilfsmittel teilgefüllt ist und dem aus einem Bioreaktor über eine im Rezirkulationskreislauf angeordnete Pumpe eine die abzutrennenden Zellen enthaltende Zelllösung zugeführt wird.

Für die Anschwemmfiltration wird ein pulverförmiges Filterhilfsmittel verwendet, welches in der Filtrationsindustrie auch unter den Synonymen Kieselgur/Kieselerde/Diatomeenerde/Diatomeceous Earth oder Diotome bekannt ist. Dieses Filterhilfsmittel hat die Eigenschaft, zusammen mit den partikulären Bestandteilen, die aus einer Flüssigkeit abgetrennt werden sollen, auf einem Trägermaterial (z.B. ein Filtervlies) einen Anschwemmkuchen zu bilden, der sich durch besonders hohe Porosität auszeichnet und eine hohe Filterkapazität ermöglicht.

Das Filterhilfsmittel bringt in Pulverform gewisse Risiken in der Handhabung mit sich und muss entsprechend sicher gehandhabt werden, wie z. B. bei der Handhabung von Pulvern auf Basis von Siliciumdioxid (z. B. quarzhaltige Stäube), bei welchen bei Inhalation von lungengängigen Partikeln das Risiko von Silikosen besteht. Dabei hat sich in der Biotechnologie der Einsatz von Einwegprodukten als besonders vorteilhaft erwiesen. Bei Einwegprodukten ist es von Vorteil, wenn diese kein Metall aufweisen und wenn sie relativ einfach und kostengünstig ausgebildet sind.

Aus dem nächstliegenden Stand der Technik der US 2004/0245124 A1 sind ein System und ein Verfahren zur Zellabtrennung über einen Filter mittels Anschwemmfiltration bekannt. Dabei wird einem in einem Rezirkulationskreislauf angeordneten Pulver- bzw. Mischbehälter, der mit einem pulverförmigen Filterhilfsmittel teilbefüllt ist, über eine im Rezirkulationskreislauf angeordnete Pumpe Zelllösung aus einem Bioreaktor zugeführt. Der nächstliegende Stand der Technik offenbart lediglich einen Kreislauf bestehend aus einer Mischkammer, einem Leitungssystem und einer Pumpe. Eine Quelle, welche zum Zuführen einer Lösung in die Mischkammer dient, ist nicht in dem Kreislauf angeordnet. Gemäß der Offenbarung des nächstliegenden Stands der Technik kann die in der Quelle enthaltene Lösung lediglich über eine Klammer dem Kreislauf hinzugefügt werden.

Nachteilig dabei ist, dass der Bioreaktor außerhalb des Rezirkulationskreislaufes angeordnet ist und der Pulverbeutel die gesamte Zelllösung bzw. Zellbrühe des Bioreaktors zusätzlich zum Filterhilfsmittel aufnehmen muss. Dies ist schwer zu handhaben und relativ teuer. Weiterhin wird zur Filtration eine dem Filter vorgelagerte zweite Pumpe benötigt.

Aufgabe der vorliegenden Erfindung ist es daher, ein Zellabtrennungssystem für die Anschwemmfiltration zu schaffen, das sicher ist, eine Kontamination der Umgebung vermeidet, einfach handhabbar ist und zudem zur kostengünstigen Verwendung als Einwegprodukt seiner relevanten Teile geeignet ist.

Weitere Aufgabe der vorliegenden Erfindung ist es, ein Verfahren zur Zellabtrennung durch Anschwemmfiltration mit einem entsprechenden System anzugeben, das sicher ist, eine Kontamination der Umgebung vermeidet, einfach handhabbar ist und zudem kostengünstig ist.

Diese Aufgabe wird durch die Gegenstände der unabhängigen Ansprüche gelöst. Bevorzugte Ausführungsformen ergeben sich aus den Unteransprüchen.

Die Aufgabe bezüglich des Zellabtrennungssystems wird in Verbindung mit dem Oberbegriff des Anspruches 1 dadurch gelöst, dass der Bioreaktor, der einen Rührer aufweist, im Rezirkulationskreislauf angeordnet ist und mit dem Pulverbeutel verbunden ist und dass ein Filter mit dem Bioreaktor über einen Konnektor verbunden ist.

Durch die Anordnung des Bioreaktors zusammen mit dem Pulverbeutel im Rezirkulationskreislauf muss nicht die gesamte Bioreaktorflüssigkeit rezirkuliert werden, um die Suspension herzustellen, sondern es wird nur ein kleiner Flüssigkeitsstrom über den Pulverbeutel mit dem Filterhilfsmittel geführt. Der Pulverbeutel ist Transport-und Mischbeutel in Einem und benötigt nicht das Volumen des Bioreaktors. Der Pulverbeutel ist kleiner und daher besser handhabbar. Es können zudem mehrere kleine Beutel mit geringen handhabbaren Kieselgurmengen von 10-15 kg als Filterhilfsmittel verwendet werden. Es können eine oder mehrere kleine gleichgroße Teilmengen, je nach Reaktorgröße und Dosiermenge, nacheinander angeschlossen und suspendiert werden, d.h. die Menge des Filterhilfsmittels bzw. die Kieselgurmenge kann unmittelbar vor der Filtration variiert werden.

Die Rezirkulation dieser kleinen Menge erfolgt nur so lange, bis die Suspension im Pulverbeutel pumpfähig ist. Danach wird die Suspension durch einen schonenden Rührer im Bioreaktor in Schwebe gehalten. Dadurch ist der Prozess schonender und minimiert die unerwünschten Einflüsse durch die Zerstörung von Zellen.

Nach einer bevorzugten Ausführungsform der Erfindung weist der Bioreaktor in vertikaler Richtung oben einen ersten Reaktorschlauch auf, der über einen ersten Konnektor mit einem in vertikaler Richtung oben am Pulverbeutel angeordneten ersten Pulverbeutelschlauch verbindbar ist. Weiterhin weist der Bioreaktor in vertikaler Richtung unten einen zweiten Reaktorschlauch auf, der über einen zweiten Konnektor mit einem in vertikaler Richtung unten am Pulverbeutel angeordneten zweiten Pulverbeutelschlauch verbindbar ist. Über die Konnektoren lassen sich die Pulverbeutel relativ einfach und schnell mit dem Bioreaktor zu dem Rezirkulationskreislauf verbinden. Damit ist es beispielsweise auch möglich, die Pulverbeutel mit den Pulverbeutelschläuchen und Konnektoren als vorsterilisierte Einheiten auszuliefern.

Gemäß einer weiteren bevorzugten Ausführungsform der Erfindung hat der erste Pulverbeutelschlauch dem ersten Konnektor zum Pulverbeutel hin ein erstes Ventil vorgelagert und der zweite Pulverbeutelschlauch dem zweiten Konnektor zum Pulverbeutel hin ein zweites Ventil vorgelagert. Der zweite Reaktorschlauch hat dem zweiten Konnektor zum Bioreaktor hin ein drittes Ventil vorgelagert.

Dadurch wird sichergestellt, dass nach dem Entfernen des Pulverbeutels eine Kontamination der Umgebung vermieden wird. Der Pulverbeutel kann mit seinen Pulverbeutelschläuchen und Ventilen entfernt oder ausgetauscht werden.

Nach einer weiteren bevorzugten Ausführungsform der Erfindung ist zwischen dem dritten Ventil und dem Bioreaktor die Pumpe angeordnet, die als eine Peristaltikpumpe ausgebildet ist. Die Anordnung macht eine weitere Pumpe für den anschließenden Filtrationsschritt unnötig. Die Ausbildung als Peristaltik- oder Schlauchpumpe führt dazu, dass sie wiederverwendet werden kann, da sie insbesondere nicht mit den Medien in Berührung kommt.

Gemäß einer weiteren bevorzugten Ausführungsform der Erfindung ist bei entferntem Pulverbeutel der Filter über einen dritten Konnektor mit dem konnektorseitigen Ende des zweiten Reaktorschlauches verbindbar.

Dadurch lässt sich die in dem Bioreaktor mit dem suspendierten Filterhilfsmittel befindliche Zelllösung einfach und sicher über die Pumpe dem Filter zur Anschwemmfiltration zuführen.

Nach einer weiteren bevorzugten Ausführungsform der Erfindung sind der Bioreaktor mit den Reaktorschläuchen, der Pulverbeutel mit den Pulverbeutelschläuchen und der Filter als Einwegteile ausgebildet. Durch die kostengünstige Ausbildung der Einwegteile wird zudem die Sicherheit zur Vermeidung einer unerwünschten Kontamination der Umwelt, z. B. durch Kontamination mit toxischen Stäuben bei der unerwünschten Freisetzung des Filterhilfsmittels, weiter erhöht.

Die Aufgabe bezüglich des Verfahrens wird in Verbindung mit dem Oberbegriff des Anspruches 9 dadurch gelöst, dass der Bioreaktor im Rezirkulationskreislauf mit dem Pulverbeutel verbunden wird, dass Zelllösung aus dem Bioreaktor in den Pulverbeutel gefördert und rezirkuliert wird, dass nach Suspendierung des Filterhilfsmittels die Förderrichtung der Pumpe umgekehrt und der Pulverbeutel entleert und bei Bedarf ausgetauscht wird, dass der Filter mit dem Bioreaktor über einen Konnektor verbunden wird, dass eine Suspension aus Zelllösung und Filterhilfsmittel aus dem Bioreaktor mittels der Pumpe dem Filter zugeführt wird, und dass die Suspension aus Zelllösung und Filterhilfsmittel aus dem Bioreaktor durch den Filter einer Filtration unterzogen wird.

Durch die Anordnung des Bioreaktors zusammen mit dem Pulverbeutel im Rezirkulationskreislauf muss nicht die gesamte Bioreaktorflüssigkeit rezirkuliert werden, um die Suspension herzustellen, sondern es wird nur ein kleiner Flüssigkeitsstrom über den Pulverbeutel mit dem Filterhilfsmittel geführt. Der Pulverbeutel ist Transport- und Mischbeutel in Einem und benötigt nicht das Volumen des Bioreaktors. Der Pulverbeutel ist kleiner und daher besser handhabbar. Es können zudem mehrere kleine Beutel, mit geringen handhabbaren Kieselgurmengen von beispielsweise 10-15 kg als Filterhilfsmittel verwendet werden.

Die Rezirkulation dieser kleinen Menge erfolgt nur so lange, bis die Suspension im Pulverbeutel pumpfähig ist. Danach wird die Suspension durch einen schonenden Rührer im Bioreaktor in Schwebe gehalten. Dadurch ist der Prozess schonender und minimiert die unerwünschten Einflüsse durch die Zerstörung von Zellen. Die Verbindung zwischen Filter und Bioreaktor kann zum einen bei entferntem Pulverbeutel erfolgen und zum anderen ist es auch möglich, den Filter über eine Abzweigung und ein weiteres Ventil mit dem Bioreaktor zu verbinden. Wichtig ist, dass nach der Entleerung des Pulverbeutels seine benachbarten Ventile geschlossen und der Rezirkulationskreislauf unterbrochen wird.

Gemäß einer weiteren bevorzugten Ausführungsform der Erfindung werden in Abhängigkeit von der benötigten Menge des Filterhilfsmittels vor dem Filtern weitere Pulverbeutel im Austausch eingesetzt und ihr suspendiertes Pulver wird dem Bioreaktor zugeführt.

Es können somit eine oder mehrere kleine gleichgroße Teilmengen, je nach Reaktorgröße und Dosiermenge, nacheinander angeschlossen und suspendiert werden. D.h. die Menge des Filterhilfsmittels bzw. die Kieselgurmenge kann unmittelbar vor der Filtration variiert werden

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:
- Figur 1:: ein Zellabtrennungssystem mit installiertem Pulverbeutel im Rezirkulationskreislauf mit geschlossenem ersten und zweiten Ventil und getrenntem Filter,
- Figur 2:: das System von Fig. 1 mit geöffneten Ventilen und Förderung von Zelllösung über eine Pumpe in den trockenen Pulverbeutel,
- Figur 3:: das System von Fig. 2 mit Förderung von in Zelllösung suspendiertem Filterhilfsmittel aus dem Pulverbeutel mit umgekehrter Förderrichtung der Pumpe in den Bioreaktor,
- Figur 4:: das System von Fig. 3 mit geschlossenen Ventilen und einem zweiten auszutauschenden Pulverbeutel mit Pulverbeutelschläuchen, Ventilen, Drucksensoren und Konnektorteilen,
- Figur 5:: das System von Fig. 4 mit geschlossenen Ventilen, mit gelöster Konnektorverbindung zwischen zweitem Pulverbeutelschlauch und zweitem Reaktorschlauch, und mit Verbindung des zweiten Reaktorschlauches über eine dritte Konnektorverbindung mit einem Zuleitungsschlauch zum Filter für die Anschwemmfiltration,
- Figur 6:: das System von Fig. 5 mit geöffneten Ventilen und Förderung von Zelllösung mit suspendiertem Filterhilfsmittel über die Pumpe zum Filter und
- Figur 7:: ein weiteres System zur Zellabtrennung mit installiertem Pulverbeutel im Rezirkulationskreislauf mit über einen Abzweig und ein zusätzliches Ventil angeschlossenem Filter.

### Beschreibung der Ausführungsbeispiele

Ein Zellabtrennungssystem 1 für die Anschwemmfiltration besteht im Wesentlichen aus einem Bioreaktor 2, einem Pulverbeutel 3, einem Filter 4 und einer Pumpe 5.

Der Bioreaktor 2 weist an seinem in vertikaler Richtung oberen Ende einen ersten Reaktorschlauch 6 und an seinem in vertikaler Richtung unteren Ende eine zweiten Reaktorschlauch 7 auf. Im Innenraum des Bioreaktors 2 ist in bekannter Weise ein Rührer 8 angeordnet. Der Bioreaktor 2 weist eine abzutrennende Zellen enthaltende Zelllösung 9 auf.

Der Pulverbeutel 3 weist in vertikaler Richtung oben einen ersten Pulverbeutelschlauch 10 auf. In vertikaler Richtung unten ist an dem Pulverbeutel 3 ein zweiter Pulverbeutelschlauch 11 angeordnet. Bei Lieferung befindet sich in dem Pulverbeutel 3 ein trockenes pulverförmiges Filterhilfsmittel 12, das für eine Anschwemmfiltration im Filter 4 benötigt wird.

Der erste Reaktorschlauch 6 und der erste Pulverbeutelschlauch 10 werden über einen ersten Konnektor 13, der beispielsweise als ein zweiteiliger Sterilverbinder ausgebildet ist, miteinander verbunden. Der erste Pulverbeutelschlauch 10 weist zwischen dem ersten Konnektor 13 und dem Pulverbeutel 3 ein erstes Ventil 14 auf. Zwischen dem ersten Ventil 14 und dem Pulverbeutel 3 ist ein erster Drucksensor 15 angeordnet. Der zweite Reaktorschlauch 7 ist mit dem zweiten Pulverbeutelschlauch 11 über einen zweiten Konnektor 16 verbunden. Zwischen dem zweiten Konnektor 16 und dem Pulverbeutel 3 weist der zweite Pulverbeutelschlauch 11 ein zweites Ventil 17 auf. Zwischen dem zweiten Ventil 17 und dem Pulverbeutel 3 ist ein zweiter Drucksensor 18 angeordnet. Der Pulverbeutel 3 mit den Pulverbeutelschläuchen 10, 11, den Ventilen 14, 17, den Drucksensoren 15, 18 und Konnektoranschlüssen 13, 16 bilden ein steril lieferbares, austauschbares Pulverbeutelset 19, das aus Einwegteilen ausgebildet ist und damit zum Einmalgebrauch (single use) geeignet ist.

Der zweite Reaktorschlauch 7 weist zwischen dem zweiten Konnektor 16 und dem Bioreaktor 2 ein drittes Ventil 20 auf. Zwischen dem dritten Ventil 20 und dem Bioreaktor 2 ist die Pumpe 5 angeordnet. Die Pumpe 5 ist beispielsweise als eine Peristaltikpumpe, die eine Schlauchpumpe sein kann, ausgebildet.
Der Bioreaktor 2 mit seinem Rührer 8 und den Reaktorschläuchen 6, 7, dem dritten Ventil 20 und den Konnektorteilen 13, 16 kann ein Reaktorset 21 bilden, das ebenfalls aus Einwegteilen ausgebildet und zum Einmalgebrauch (single use) geeignet ist. Auch das Reaktorset 21 kann steril geliefert werden.

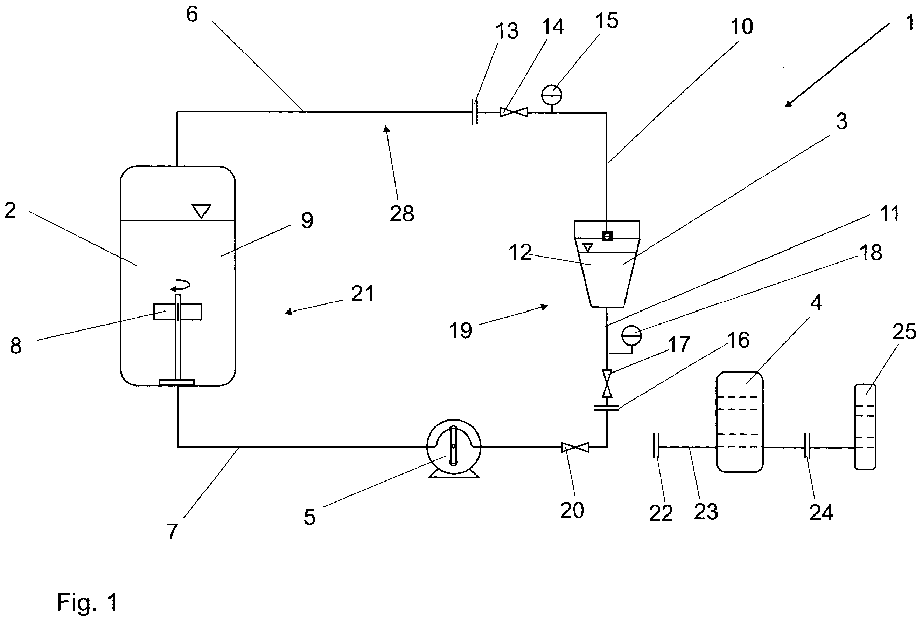

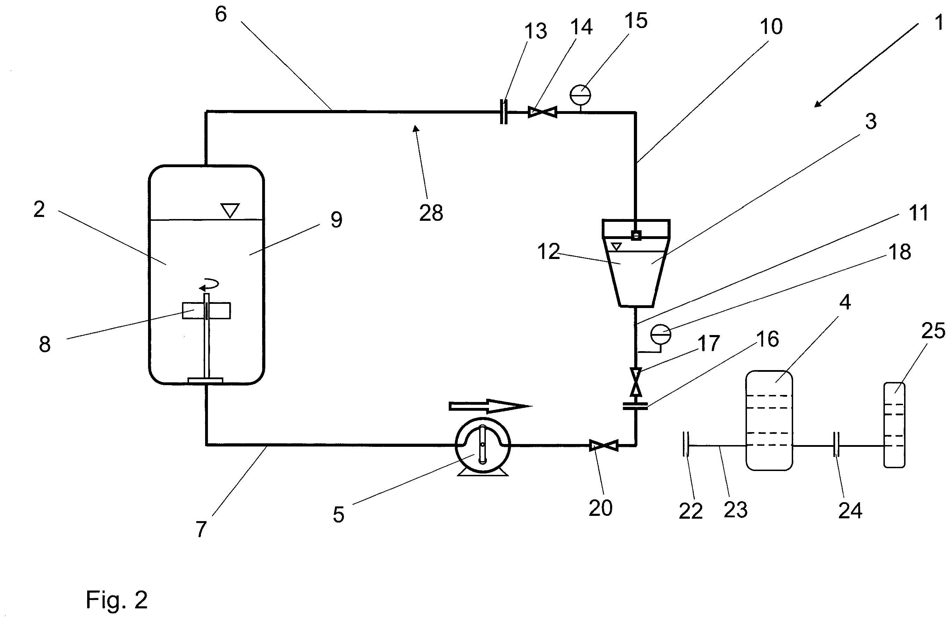

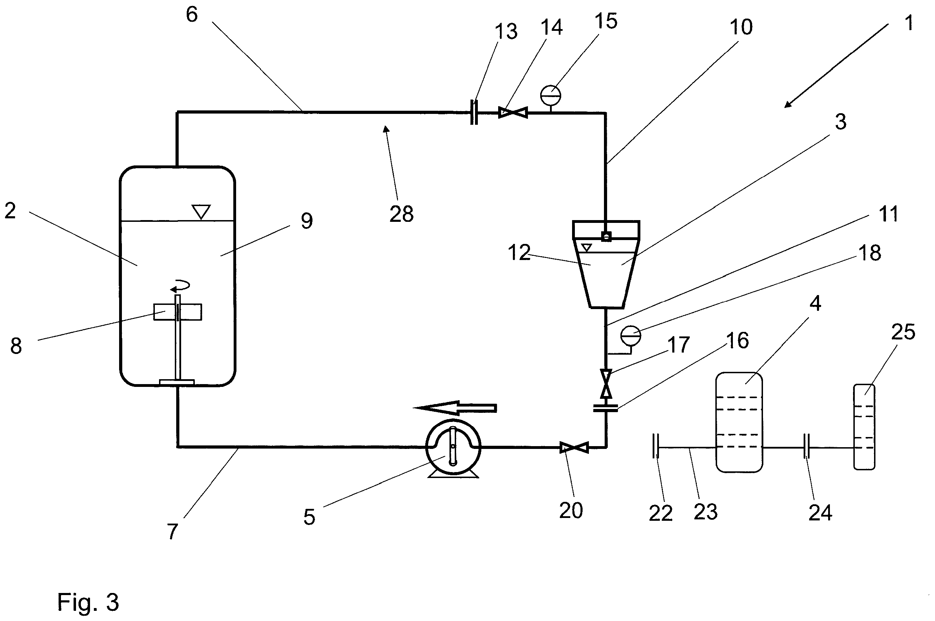

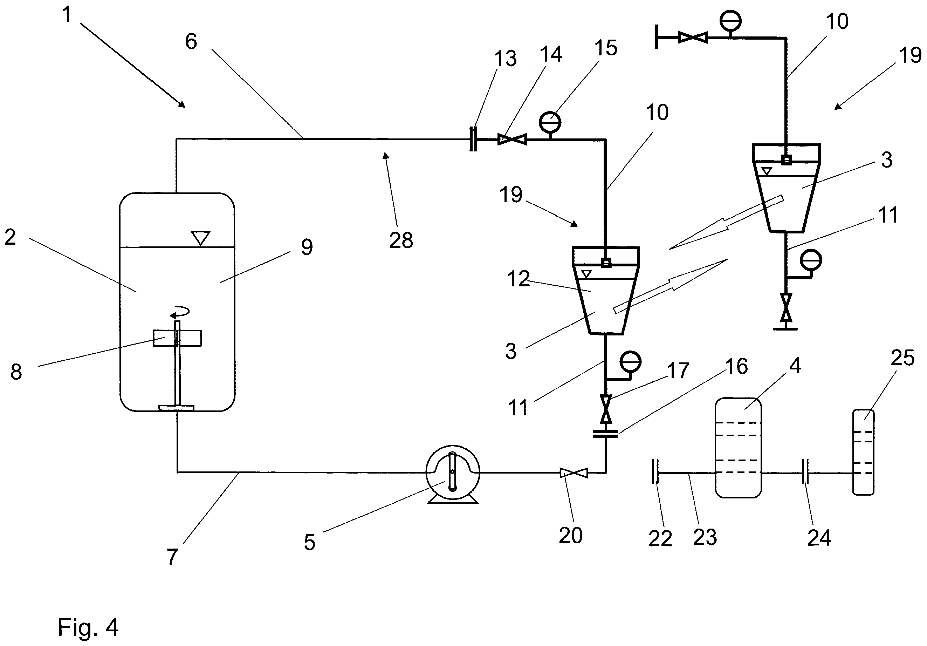

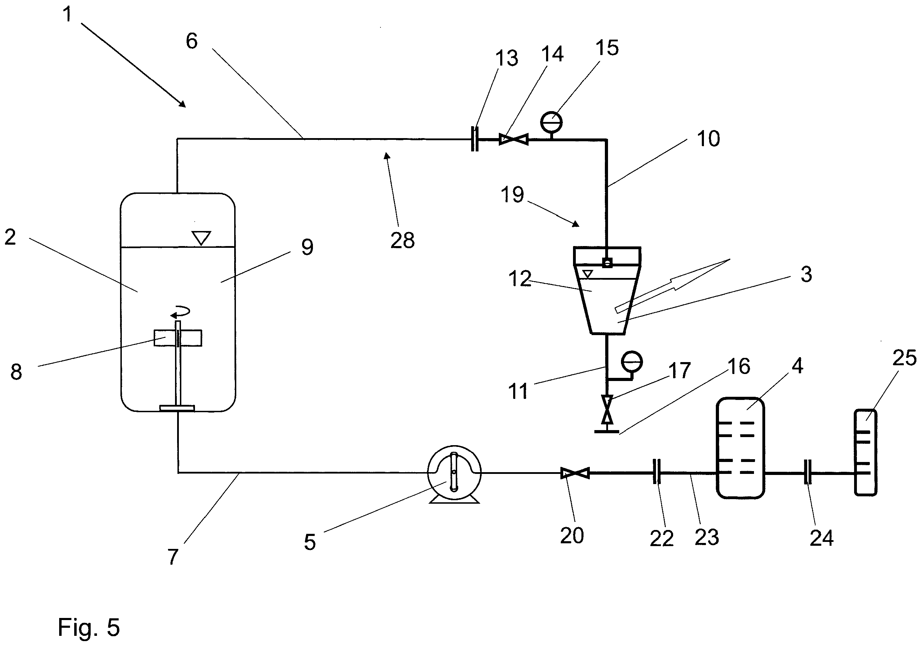

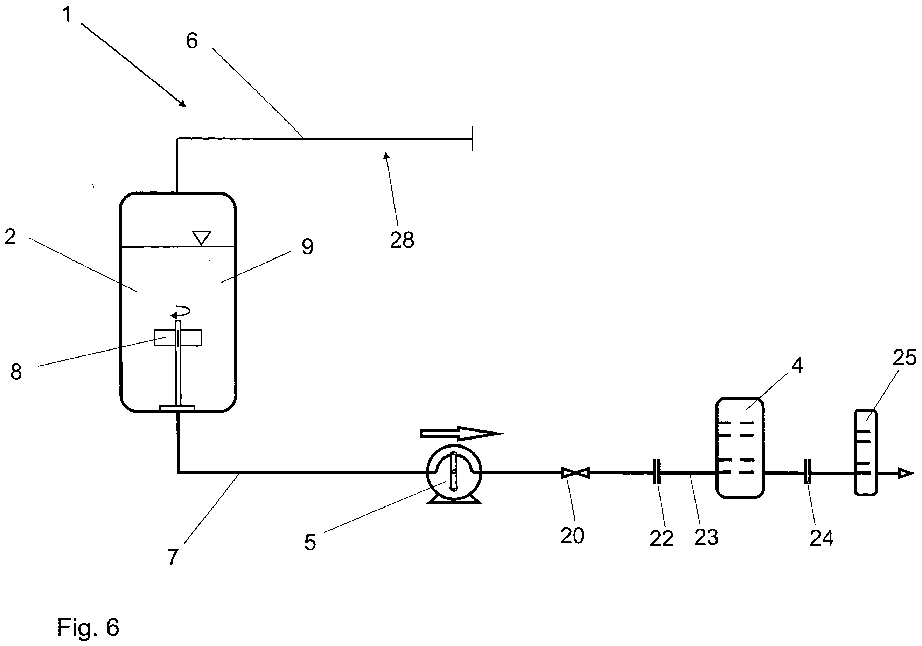

Gemäß den Ausführungsformen der Figuren 1 bis 6 wird nach dem Entfernen des Pulverbeutelsets 19 der Filter 4 über einen dritten Konnektor 22, der über einen Filterschlauch 23 mit dem Filter 4 verbunden ist, mit dem zweiten Reaktorschlauch 7 verbunden. Dem Filter 4 kann über einen vierten Konnektor 24 ein Schichten- oder Sterilfilter 25 nachgeschaltet werden.

Auch die Filter 4 und 25 können als Einwegteile ausgebildet sein.

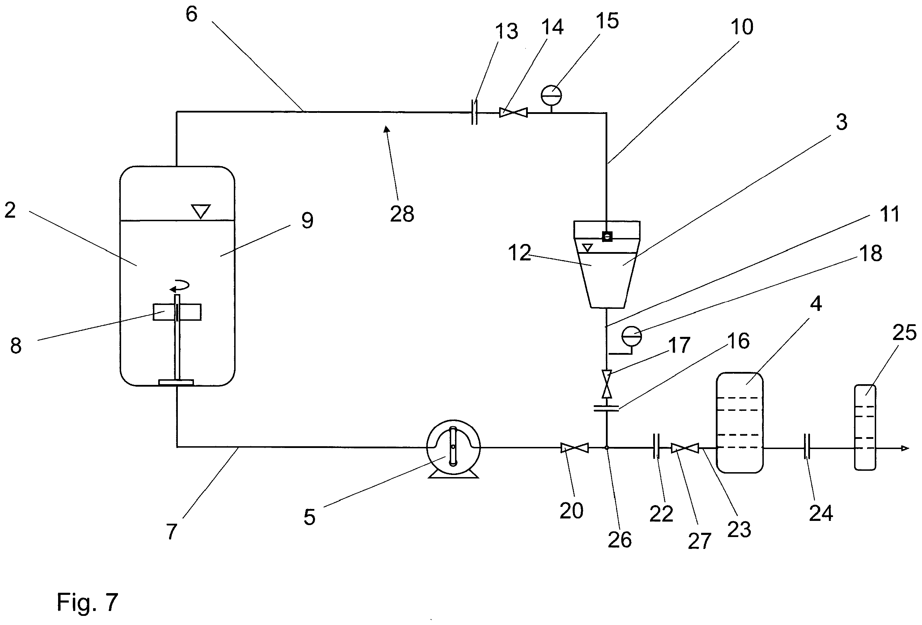

Entsprechend dem Ausführungsbeispiel der Figur 7 kann der Filter 4 auch über ein Abzweigteil 26 mit dem zweiten Pulverbeutelschlauch 11 verbunden sein. In diesem Fall ist zwischen dem dritten Konnektor 22 und dem Filter 4 im Filterschlauch 23 ein viertes Ventil 27 angeordnet.

Das Verfahren zur Zellabtrennung kann mit folgenden Prozessschritten durchgeführt werden:
1) Installation des Pulverbeutels 3 und Herstellen eines Rezirkulationskreislaufes 28 durch Verbinden des ersten Pulverbeutelschlauches 10 über den ersten Konnektor 13 mit dem ersten Reaktorschlauch 6 und Verbinden des zweiten Pulverbeutelschlauches 11 über den zweiten Konnektor 16 mit dem zweiten Reaktorschlauch 7.
2) Öffnen der Ventile 14, 17, 20, Pumpe 5 starten und Zelllösung 9 aus dem Bioreaktor 2 in den trockenen Pulverbeutel 3 fördern, so dass die Zelllösung 9 in dem Pulverbeutel 3 aufsteigt und das Filterhilfsmittel 12 benetzt und (teil-)suspendiert, wobei der Eingangs- und Ausgangsdruck am Pulverbeutel 3 über die Drucksensoren 15, 18 überwacht und gegebenenfalls korrigiert wird. Nach einer Rezirkulationszeit ist das Filterhilfsmittel 12 in der Zelllösung 9 ausreichend suspendiert und damit pumpfähig, so dass die Pumpe 5 gestoppt wird.
3) Die Pumpe wird in entgegengesetzter Förderrichtung gestartet und die pumpfähige Suspension von Zelllösung 9 und Filterhilfsmittel 12 wird vollständig aus dem Pulverbeutel 3 in den Bioreaktor 2 gefördert. Dabei entleert sich der Pulverbeutel 3 und saugt sich zusammen. Nach vollständiger Entleerung des Pulverbeutels 3 wird die Pumpe 5 gestoppt.
4) Die Ventile 14, 17, 20 werden geschlossen und das Pulverbeutelset 19 wird an den Konnektoren 13, 16 getrennt und gegebenenfalls gegen ein neues Pulverbeutelset 19 ausgetauscht.
   Die Schritte 1 bis 3 werden so oft wiederholt, bis die gesamte vorgesehene Menge an Filterhilfsmittel 12 suspendiert und in den Bioreaktor 2 eingebracht ist.
5) Das letzte Pulverbeutelset 19 wird dann an den Konnektoren 13, 16 nach dem Schließen der Ventile 14, 17, 20 gelöst und entfernt. Anschließend wird der zweite Reaktorschlauch 7 über den dritten Konnektor 22 mit dem Filter 4 verbunden. Alternativ kann der Filter 4 nach Schließen des Ventils 17 auch über das vierte Ventil 27 und den dritten Konnektor 22 mit dem Abzweigteil 26 und damit mit dem zweiten Reaktorschlauch 7 verbunden werden.
6) Die Pumpe 5 wird mit Förderrichtung zum Filter 4 hin gestartet und die Zelllösung 9 mit suspendiertem Filterhilfsmittel 12 wird aus dem Bioreaktor 2 durch die Filter 4, 25 gefördert.

Besonders geeignet für die Durchführung des erfindungsgemäßen Verfahrens ist ein Pulverbeutel 3, dessen vertikal nach unten weisendes und mit dem zweiten Pulverbeutelschlauch 11 verbundenes Ende trichterförmig bzw. konisch verjüngt ist und der mit einer externen, nicht gezeigten Schütteleinrichtung verbindbar ist, die auf das Äußere des Pulverbeutels 3 einwirkt, um eine effektive Suspendierung des Filterhilfsmittels 12 in der in dem Pulverbeutel 3 befindlichen Zelllösung 9 zu bewirken.

## Patentansprüche

1. Zellabtrennungssystem (1) umfassend einen Filter (4), einen Bioreaktor (2), der einen Rührer (8) aufweist, und einen eine Pumpe (5) umfassenden Rezirkulationskreislauf (28), in dem ein Pulverbeutel (3) angeordnet ist, der mit einem pulverförmigen Filterhilfsmittel (12) teilgefüllt ist und dem aus dem Bioreaktor (2) über die Pumpe (5) eine die abzutrennenden Zellen enthaltende Zelllösung (9) zuführbar ist,
**dadurch gekennzeichnet,**
**dass** der Bioreaktor (2) im Rezirkulationskreislauf (28) angeordnet und mit dem Pulverbeutel (3) verbunden ist und
**dass** der Filter (4) mit dem Bioreaktor (2) über einen Konnektor (13, 16, 22) verbunden ist.

2. Zellabtrennungssystem nach Anspruch 1,
**dadurch gekennzeichnet,**
**dass** der Bioreaktor (2) in vertikaler Richtung oben einen ersten Reaktorschlauch (6) aufweist, der über einen ersten Konnektor (13) mit einem in vertikaler Richtung oben am Pulverbeutel (3) angeordneten ersten Pulverbeutelschlauch (10) verbindbar ist, und
**dass** der Bioreaktor (2) in vertikaler Richtung unten einen zweiten Reaktorschlauch (7) aufweist, der über einen zweiten Konnektor (16) mit einem in vertikaler Richtung unten am Pulverbeutel (3) angeordneten zweiten Pulverbeutelschlauch (11) verbindbar ist.

3. Zellabtrennungssystem nach Anspruch 2,
**dadurch gekennzeichnet,**
**dass** der erste Pulverbeutelschlauch (10) dem ersten Konnektor (13) zum Pulverbeutel (3) hin ein erstes Ventil (14) vorgelagert hat,
**dass** der zweite Pulverbeutelschlauch (11) dem zweiten Konnektor (16) zum Pulverbeutel (3) hin ein zweites Ventil (17) vorgelagert hat, und
**dass** der zweite Reaktorschlauch (7) dem zweiten Konnektor (16) zum Bioreaktor (2) hin ein drittes Ventil (20) vorgelagert hat.

4. Zellabtrennungssystem nach Anspruch 3,
**dadurch gekennzeichnet,**
**dass** zwischen dem dritten Ventil (20) und dem Bioreaktor (2) die Pumpe (5) angeordnet ist.

5. Zellabtrennungssystem nach Anspruch 4,
**dadurch gekennzeichnet,**
**dass** die Pumpe (5) als eine Peristaltikpumpe ausgebildet ist.

6. Zellabtrennungssystem nach einem der Ansprüche 3 bis 5,
**dadurch gekennzeichnet,**
**dass** der Pulverbeutel (3) mit seinen Pulverbeutelschläuchen (10, 11) und Ventilen (14, 17) entfernbar oder austauschbar ist.

7. Zellabtrennungssystem nach einem der Ansprüche 3 bis 6,
**dadurch gekennzeichnet,**
**dass** der Filter (4) über einen dritten Konnektor (22) mit dem konnektorseitigen Ende des zweiten Reaktorschlauches (7) verbindbar ist.

8. Zellabtrennungssystem nach einem der Ansprüche 1 bis 7,
**dadurch gekennzeichnet,**
**dass** der Bioreaktor (2) mit den Reaktorschläuchen (6, 7), der Pulverbeutel (3) mit den Pulverbeutelschläuchen (10, 11) und der Filter (4) als Einwegteile ausgebildet sind.

9. Verfahren zur Zellabtrennung über einen Filter (4) mit einem in einem Rezirkulationskreislauf (28) angeordneten Pulverbeutel (3), der mit einem pulverförmigen Filterhilfsmittel (12) teilgefüllt ist und dem aus einem Bioreaktor (2) über eine im Rezirkulationskreislauf (28) angeordnete Pumpe (5) eine die abzutrennenden Zellen enthaltende Zelllösung (9) zugeführt wird,
**dadurch gekennzeichnet,**
**dass** der Bioreaktor (2) im Rezirkulationskreislauf (28) mit dem Pulverbeutel (3) verbunden wird,
**dass** Zelllösung (9) aus dem Bioreaktor (2) in den Pulverbeutel (3) gefördert und rezirkuliert wird,
**dass** nach Suspendierung des Filterhilfsmittels (12) die Förderrichtung der Pumpe (5) umgekehrt und der Pulverbeutel (3) entleert wird,
**dass** der Filter (4) mit dem Bioreaktor (2) über einen Konnektor (13, 16, 22) verbunden wird,
**dass** eine Suspension aus Zelllösung (9) und Filterhilfsmittel (12) aus dem Bioreaktor (2) mittels der Pumpe (5) dem Filter (4) zugeführt wird, und
**dass** die Suspension aus Zelllösung (9) und Filterhilfsmittel (12) aus dem Bioreaktor (2) durch den Filter (4) einer Filtration unterzogen wird.

10. Verfahren nach Anspruch 9,
**dadurch gekennzeichnet,**
**dass** in Abhängigkeit von der benötigten Menge des Filterhilfsmittels (12) vor dem Filtern weitere Pulverbeutel (3) im Austausch eingesetzt werden und ihr suspendiertes Pulver dem Bioreaktor (2) zugeführt wird.

11. Verfahren nach Anspruch 9 oder 10,
**dadurch gekennzeichnet,**
**dass** das Filterhilfsmittel (12) im Bioreaktor (2) durch einen Rührer (8) in Schwebe gehalten wird.

## Claims

1. A system (1) for cell separation comprising a filter (4), a bioreactor (2), which has an agitator (8), and a recirculation circuit (28) comprising a pump (5), in which a powder bag (3) is arranged which is partly filled with a pulverulent filter aid (12) and to which a cell solution (9) containing the cells to be separated can be delivered from the bioreactor (2) via the pump (5),
**characterized in**
**that** the bioreactor (2) is arranged in the recirculation circuit (28) and is connected to the powder bag (3) and
**that** the filter (4) is connected to the bioreactor (2) via a connector (13, 16, 22).

2. The system according to claim 1,
**characterized in**
**that** the bioreactor (2) has on the top in a vertical direction a first reactor hose (6) that is connectable via a first connector (13) to a first powder bag hose (10) arranged on the top of the powder bag (3) in a vertical direction, and
**that** the bioreactor (2) has at a bottom in a vertical direction a second reactor hose (7) that is connectable via a second connector (16) to a second powder bag hose (11) arranged at the bottom of the powder bag (3) in a vertical direction.

3. The system according to claim 2,
**characterized in**
**that** the first powder bag hose (10) has, preceding the first connector (13) toward the powder bag (3), a first valve (14),
**that** the second powder bag hose (11) has, preceding the second connector (16) toward the powder bag (3), a second valve (17), and
**that** the second reactor hose (7) has, preceding the second connector (16) toward the bioreactor (2), a third valve (20) .

4. The system according to claim 3,
**characterized in**
**that** the pump (5) is arranged between the third valve (20) and the bioreactor (2).

5. The system according to claim 4,
**characterized in**
**that** the pump (5) is a peristaltic pump.

6. The system according to any of claims 3 to 5, **characterized in**
**that** the powder bag (3) with its powder bag hoses (10, 11) and with valves (14, 17), is removable or exchangeable.

7. The system according to any of claims 3 to 6,
**characterized in**
**that** the filter (4) is connectable via a third connector (22) to a connector-side end of the second reactor hose (7).

8. The system according to any of claims 1 to 7,
**characterized in**
**that** the bioreactor (2) with the reactor hoses (6, 7), the powder bag (3) with the powder bag hoses (10, 11) and the filter (4) are designed as disposable parts.

9. A method for cell separation via a filter (4) with a powder bag (3) arranged in a recirculation circuit (28), which is partly filled with a pulverulent filter aid (12) and to which a cell solution (9) containing the cells to be separated can be delivered from a bioreactor (2) via a pump (5) arranged in the recirculation circuit (28),
**characterized in**
**that** the bioreactor (2) in the recirculation circuit (28) is connected to the powder bag (3),
**that** the cell solution (9) is conveyed from the bioreactor (2) to the powder bag (3) and recirculated,
**that** after suspension of the filter aid (12) the conveyor direction of the pump (5) is reversed and the powder bag (3) is emptied,
**that** the filter (4) is connected to the bioreactor (2) via a connector (13, 16, 22),
**that** a suspension composed of the cell solution (9) and the filter aid (12) from the bioreactor (2) is supplied to the filter (4) by means of the pump (5), and
**that** the suspension composed of the cell solution (9) and the filter aid (12) from the bioreactor (2) is subjected to filtration by means of the filter (4).

10. The method according to claim 9,
**characterized in**
**that**, depending on the required quantity of filter aid (12), further powder bags (3) are used in exchange before filtration and their suspended powder is delivered to the bioreactor (2).

11. The method according to claim 9 or 10,
**characterized in**
**that** an agitator (8) holds the filter aid (12) suspension in the bioreactor (2).

## Revendications

1. Système de séparation de cellules (1) comprenant un filtre (4), un bioréacteur (2), qui présente un agitateur (8), et un circuit de recirculation (28) comprenant une pompe (5), dans lequel un sachet de poudre (3) est disposé, qui est partiellement rempli d'un adjuvant de filtration (12) pulvérulent et auquel une solution de cellules (9) contenant les cellules à séparer peut être amenée à partir du bioréacteur (2) par l'intermédiaire de la pompe (5),
**caractérisé en ce**
**que** le bioréacteur (2) est disposé dans le circuit de recirculation (28) et relié au sachet de poudre (3) et
**que** le filtre (4) est relié au bioréacteur (2) par l'intermédiaire d'un connecteur (13, 16, 22).

2. Système de séparation de cellules selon la revendication 1,
**caractérisé en ce**
**que** le bioréacteur (2) présente dans la direction verticale en haut un premier tuyau de réacteur (6), qui peut être relié à un premier tuyau de sachet de poudre (10) disposé dans la direction verticale en haut sur le sachet de poudre (3) par l'intermédiaire d'un premier connecteur (13), et
**que** le bioréacteur (2) présente dans la direction verticale en bas un deuxième tuyau de réacteur (7), qui peut être relié à un deuxième tuyau de sachet de poudre (11) disposé dans la direction verticale en bas sur le sachet de poudre (3) par l'intermédiaire d'un deuxième connecteur (16).

3. Système de séparation de cellules selon la revendication 2,
**caractérisé en ce**
**que** le premier tuyau de sachet de poudre (10) présente montée en amont du premier connecteur (13) en direction du sachet de poudre (3) une première soupape (14),
**que** le deuxième tuyau de sachet de poudre (11) présente montée en amont du deuxième connecteur (16) en direction du sachet de poudre (3) une deuxième soupape (17), et
**que** le deuxième tuyau de réacteur (7) présente montée en amont du deuxième connecteur (16) en direction du bioréacteur (2) une troisième soupape (20).

4. Système de séparation de cellules selon la revendication 3,
**caractérisé en ce**
**que** la pompe (5) est disposée entre la troisième soupape (20) et le bioréacteur (2).

5. Système de séparation de cellules selon la revendication 4,
**caractérisé en ce**
**que** la pompe (5) est réalisée sous la forme d'une pompe péristaltique.

6. Système de séparation de cellules selon l'une quelconque des revendications 3 à 5,
**caractérisé en ce**
**que** le sachet de poudre (3) avec ses tuyaux de sachet de poudre (10, 11) et soupapes (14, 17) peut être retiré ou échangé.

7. Système de séparation de cellules selon l'une quelconque des revendications 3 à 6,
**caractérisé en ce**
**que** le filtre (4) peut être relié à l'extrémité côté connecteur du deuxième tuyau de réacteur (7) par l'intermédiaire d'un troisième connecteur (22).

8. Système de séparation de cellules selon l'une quelconque des revendications 1 à 7,
**caractérisé en ce**
**que** le bioréacteur (2) avec les tuyaux de réacteur (6, 7), le sachet de poudre (3) avec les tuyaux de sachet de poudre (10, 11) et le filtre (4) sont réalisés sous la forme de pièces à usage unique.

9. Procédé pour la séparation de cellules par l'intermédiaire d'un filtre (4) avec un sachet de poudre (3), disposé dans un circuit de recirculation (28), qui est partiellement rempli d'un adjuvant de filtration (12) pulvérulent et auquel une solution de cellules (9) contenant les cellules à séparer peut être amenée à partir d'un bioréacteur (2) par l'intermédiaire d'une pompe (5) disposée dans le circuit de recirculation (28),
**caractérisé en ce**
**que** le bioréacteur (2) dans le circuit de recirculation (28) est relié au sachet de poudre (3),
**que** la solution de cellules (9) provenant du bioréacteur (2) est refoulée dans le sachet de poudre (3) et amenée en recirculation,
**qu'**après suspension de l'adjuvant de filtration (12), la direction de refoulement de la pompe (5) est inversée et le sachet de poudre (3) est vidé,
**que** le filtre (4) est relié au bioréacteur (2) par l'intermédiaire d'un connecteur (13, 16, 22),
**qu'**une suspension composée de la solution de cellules (9) et de l'adjuvant de filtration (12) est amenée à partir du bioréacteur (2) au filtre (4) au moyen de la pompe (5), et
**que** la suspension composée de la solution de cellules (9) et de l'adjuvant de filtration (12) provenant du bioréacteur (2) est soumise à une filtration à travers le filtre (4).

10. Procédé selon la revendication 9,
**caractérisé en ce**
**qu'**en fonction de la quantité nécessaire de l'adjuvant de filtration (12), d'autres sachets de poudre (3) sont insérés en échange avant la filtration et leur poudre en suspension est amenée au bioréacteur (2).

11. Procédé selon la revendication 9 ou 10,
**caractérisé en ce**
**que** l'adjuvant de filtration (12) est maintenu en suspension dans le bioréacteur (2) par un agitateur (8).
